# EUROPÄISCHE PATENTSCHRIFT

(11) **EP 1 001 799 B1**
(45) Veröffentlichungstag und Bekanntmachung des Hinweises auf die Patenterteilung: **31.10.2001**
(21) Anmeldenummer: 98929194.3
(22) Anmeldetag: 07.07.1998
(51) Int. Cl.: A61K 38/06, A61K 38/07, A61K 38/08, A61K 47/48

(54) **PHARMAZEUTISCHE ZUSAMMENSETZUNG ENTHALTEND PEPTICHEMIO**
PHARMACEUTICAL COMPOSITION CONTAINING PEPTICHEMIO
COMPOSITION PHARMACEUTIQUE CONTENANT DU PEPTICHEMIO

(30) Priorität: 07.07.1997 CH 165197
(43) Veröffentlichungstag der Anmeldung: 24.05.2000
(60) Teilanmeldung: 01201272.0
(73) Patentinhaber: PTC Pharma AG, 3000 Bern 25 (CH)
(72) Erfinder: MEHLEM, Francesco, CH-3048 Worblaufen (CH)
(74) Vertreter: BOVARD AG - Patentanwälte
(86) Internationale Anmeldenummer: CH9800300
(87) Internationale Veröffentlichungsnummer: WO9902177

(56) Entgegenhaltungen:
- WO-A-94/20136
- FR-A- 2 094 175
- FR-A- 2 101 226
- RAJEWSKI ET AL.: "Pharmaceutical applications of cyclodextrins. 2. In vivo drug delivery" JOURNAL OF PHARMACEUTICAL SCIENCES, Bd. 85, Nr. 11, November 1996, Seiten 1142-1168, XP000629515
- ASTALDI : "Peptichemio: a multifaceted antiblastic drug" WADLEY MEDICAL BULLETIN, Bd. 5, Nr. 3, 1975, Seiten 303-326, XP002079526

## Beschreibung

Die vorliegende Erfindung bezieht sich auf eine pharmazeutische Zusammensetzung, in welcher pharmazeutisch aktive Peptide, die L-m-Sarcolysin als Aminosäurebaustein enthalten, formuliert sind. Die Wirkstoffe dienen insbesondere zur Chemotherapie gegen Krebsleiden und werden speziell gegen Melanome eingesetzt. Eine Trägersubstanz auf Basis von Cyclodextrin dient einer verzögerten Freisetzung der Wirkstoffe und ist für eine genügende Bioverfügbarkeit während einer ausreichend langen Zeitdauer verantwortlich.

Ein Komplex von sechs Peptiden, die m-L-Sarcolysin enthalten, ist unter dem Warennamen "Peptichemio" (Istituto Sieroterapico Milanese S. Belfanti, Milano, IT) für die Chemotherapie gegen Krebs bekannt geworden. Es wurde gefunden, dass die Aktivität der einzelnen Peptide verschieden ist und dass besonders ein Vertreter eine sehr hohe Toxizität für Melanomzellen aufweist. Die Peptide sind eine Entwicklung, welche mit dem Produkt "Melphalan", d.h. 4-[bis(2-Chloroethyl)]-amino-L-phenylalanin begonnen hat. Es wurde gefunden, dass dieses Produkt eine zytostatische Wirkung hat und sowohl für die Myelom- als auch für die Melanomtherapie eingesetzt werden kann. Zur Weiterentwicklung des Wirkstoffes wurden Derivate des Produktes hergestellt. Daraus resultierte auch das L-m-Sarcolysin, das weiter deriviert wurde, indem Peptide hergestellt wurden, welche die modifizierte Aminosäure als Baustein enthielten. Eine Kombination von 6 derartigen Oligopeptiden bildete das aktive Prinzip der Antitumormittel "Peptichemio". Die 6 Peptide sind folgende:
- L-Seryl-L-p-fluorphenylalanyl-L-m-sarcolysyl-ethylester
- L-Prolyl-L-m-sarcolysyl-L-p-fluorphenylalanin-ethylester
- L-m-Sarcolysyl-N-nitro-L-arginyl-L-norvalin-ethylester
- L-p-Fluorphenylalanyl-L-m-sarcolysyl-L-asparagin-ethylester
- L-p-Fluorphenylalanyl-glycyl-L-m-sarcolysyl-norvalin-ethyfeste /r
- L-m-Sarcolysyl-L-arginyl-L-lysyl-L-m-sarcolysyl-L-histidinmethylester

Es wurde gefunden, dass Peptichemio weniger toxisch gegen humane Lymphoplasten ist als m-L-Sarcolysin allein. Zusätzlich zur kleineren Toxizität von Peptichemio wurde eine verminderte Bildung von DNA-Vernetzungen festgestellt. Im Gegensatz dazu wurde eine erhöhte Zytotoxizität von Peptichemio gegen humane Melanomzellinien festgestellt, im Vergleich zum m-L-Sarcolysin. Hier war die höhere Zytotoxizität mit einer grösseren DNA-Vernetzung verbunden. Die Vergleichsanalyse der 6 Peptide zeigte Unterschiede in der Zytotoxizität gegen Melanomzellen. Eines der 6 Peptide zeigte eine beträchtlich höhere Zytotoxizität im Vergleich zum Peptichemio selbst (R. Levenson, et al., Radiumhemmet, Karolinska Hospital, Stockholm SE, Eur. J. Cancer Clin. Oncol.; 23: 6, 783-788, 1987). Gemäss diesen Studien wurde gefunden, dass das Peptid L-Propyl-m-sarcolysyl-L-p-fluorphenylalanin (PSF) 35 x bzw. 28 x toxischer gegen RPMI 8322 Melanomzellen war als Melphalan bzw. m-Sarcolysin. Ähnliche Unterschiede zwischen den Wirkstoffen wurden auch für andere Melanomzellinien gefunden.

Damit die in vitro gefundene Aktivität ebenfalls in vivo entfaltet werden kann, muss die Bioverfügbarkeit des Wirkstoffes im Körper ausreichend sein. Es muss eine genügend hohe Konzentration während einer genügenden Zeitdauer vorhanden sein bzw. die Halbwertszeit des aktiven Prinzipes muss genügend sein.

Es ist demzufolge Aufgabe der vorliegenden Erfindung, eine pharmazeutische Zusammensetzung zur Verfügung zu stellen, durch welche eines oder mehrere der 6 Peptide in solcher Weise verabreicht werden können, damit die Bioverfügbarkeit den gestellten Anforderungen genügt.

Es wurde gefunden, dass eine Kombination eines oder mehrerer der 6 Peptide mit einem gegebenenfalls substitutierten Cyclodextrin als Träger diese Anforderungen erfüllt.

Gegenstand der vorliegenden Erfindung ist demzufolge die im Patentanspruch 1 definierte pharmazeutische Zusammensetzung. Als Wirkstoff enthält die Zusammensetzung mindestens eines der Peptide, ausgewählt aus der Gruppe:
- L-Seryl-L-p-fluorphenylalanyl-L-m-sarcolysin
- L-Prolyl-L-m-sarcolysyl-L-p-fluorphenylalanin
- L-m-Sarcolysyl-N-nitro-L-arginyl-L-norvalin
- L-p-Fluorphenylalanyl-L-m-sarcolysyl-L-asparagin
- L-p-Fluorphenylalanyl-glycyl-L-m-sarcolysyl-norvalin
- L-m-Sarcolysyl-L-arginyl-L-lysyl-L-m-sarcolysyl-L-histidin
und ihre Niederalkylester, insbesondere ihre Ethyl- und Methylester und/oder Säureadditionssatze davon.

Die Peptide liegen vorzugsweise in Form von Hydrochloriden oder Hydrobromiden vor. Vorzugsweise wird L-Prolyl-m-sarcolysyl-L-p-fluorphenylalanin (PSF) verwendet. Der Cyclodextrinträgerstoff, welcher der Regulierung der Bioverfügbarkeit dient, ist vorzugsweise Hydroxypropyl-β-cyclodextrin. Dieses Produkt ist im Handel erhältlich und wurde beschrieben von Pitha et al. in "Hydroxypropyl-β-cyclodextrin preparation and characterization", International Journal of Pharmaceutics, 29: 73 bis 83 (1986). Hydroxypropyl-β-cyclodextrin ist unter dem Warennamen "Incapsin" der Firma Janssen im Handel erhältlich. β-Cyclodextrin wird nur für den oralen und topischen Bereich verwendet. Zur parenteralen Verabreichung wird ein substitutiertes β-Cyclodextrin, vorzugsweise Hydroxypropyl-β-cyclodextrin verwendet. Die Cyclodextrinverbindung bildet mit den Peptiden einen Komplex und bewirkt bei parenteraler Applikation, dass der Wirkstoff im Organismus in genügender Konzentration zur Verfügung gestellt wird. Vorzugsweise wird als Wirkstoff PSF zusammen mit Hydroxpropyl-ß-Cyclodextrin verwendet, wobei ein Mol-Verhältnis PSF zu Hydroxpropyl-β-Cyclodextrin im Bereich von 1:1 bis 1:10 verwendet wird. Typische Beispiele derartiger Verhältnisse sind 1:1, 1:2, 1:3, 1:4. Der Wirkstoff bildet mit dem gegebenenfalls substituierten Cyclodextrin einen Komplex, in welchem das Peptid stabilisiert wird. Die Halbwertszeiten in der Grössenordnung von 10 bis 30 min. des Wirkstoffes in einer Körperflüssigkeit werden durch Inklusion in einen Komplex mit Cyclodextrin erhöht, wobei eine Steuerung durch das obenerwähnte Verhältnis möglich ist. Der Komplex kann durch den Organismus besser aufgenommen und absorbiert werden, wobei die Bioverfügbarkeit positiv beeinflusst wird. Das Verhältnis muss der Krankheit des Patienten und seinem Gesundheitszustand angepasst werden. Das Produkt kann als festes Produkt oder als Konzentrat vorgelegt werden, welches zur Herstellung von injizierbaren Lösungen oder Infusionen dient. Falls therapeutisch erforderlich, kann die pharmazeutische Formulierung auch andere Wirkstoffe wie z.B. Chlorphenamin enthalten.

Für orale Formulierungen wird das PSF mit α-, oder -β- oder γ-Cyclodextrin vermischt. Das Verhältnis von Peptid zu Cyclodextrin beträgt hier beispielsweise 1 : 1 bis 1 : 10, typischerweise 1:1, 1:2, 1:3. 1:4. Die Kombination wird gegebenenfalls zusammen mit einem geeigneten Trägerstoff in Kapseln verpackt.

### Herstellungsbeispiel (Wirkstoff):

### Synthese von L-prolyl-L-m-sarcolysyl-L-p-fluorphenylalanin-ethylesterhydrochlorid

### a) N-Carbobenzoxy-L-m-sarcolvsyl-L-p-fluorphenylalanin-ethylester

52,5 g L-p-Fluorphenylalaninethylester Hydrochlorid werden mit 75 ml Na₂CO₃ (Natriumcarbonat) gesättigte Lösung und 150 ml CHCl₃ behandelt. Die Mischung wird ausgeschüttelt und die organische Phase wird getrennt und aufbewahrt. Die wässrige Phase wird mit 75 ml CHCl₃ ein zweites Mal ausgeschüttelt. Die vereinigten Chloroformextrakte werden gemischt und einmal mit Wasser gewaschen, und dann von der wässrigen Phase getrennt und auf wasserfreiem Na₂SO₄ getrocknet. Die Konzentration von Aminosäureester wird durch eine Titration mit HClO₄ (Perchlorsäure) bestimmt. Die Ausbeute entspricht ungefähr dem theoretischen Wert; sie liegt bei 98%.

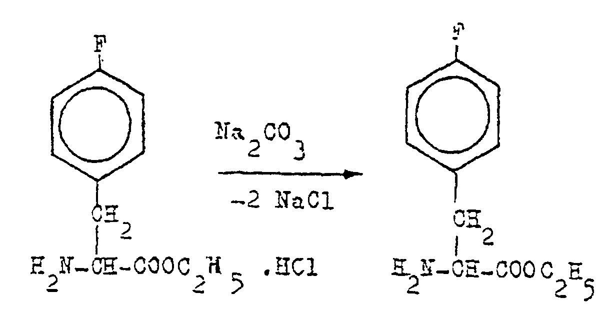

286,5 ml einer Chloroformlösung, die 0,1905 Mol L-p-Fluorphenylalaninethylester enthält, werden mit 83,7 g (0,1905 Mole) N-Cbzo-L-m-sarcolysin versetzt. Die Lösung wird auf einem Eisbad gekühlt.

Der gekühlten Lösung werden unter Rühren 41,25 g (0,200 Mol Dicyclohexylcarbodiimid - DCC) und 60 ml Chloroform dazugegeben, wobei die Lösung während 30 min. unter gleichzeitiger Kühlung ständig gerührt wird. Unter Umständen kann die Mischung zu fester Masse erstarren. In diesem Fall wird die Masse durch Zugabe von 150 ml Chloroform wieder flüssig gemacht, wobei sie unter leichtem Erwärmen gerührt wird. Auf diese Weise wird die Auflösung des ausgefallenen Produktes beschleunigt. Die Reaktion ist 2 h nach Zugabe des DDC beendet. Das Reaktionsende wird durch TLC-Kontrolle festgestellt (Dünnschochtchromatographie; Kielgel G-Schicht, Lösungsmittel: Chloroform + Aceton 9:1, Sichtbarmachung durch Besprühen mit verdünnter, saurer KMnO₄-Lösung). Der ausgefallene Dicyclohexylharnstoff wird durch Filtration abgetrennt. Die Lösung wird zuerst mit wenig Wasser, dann mit gesättigter Na₂CO₃-Lösung gewaschen. Die Chloroformlösung wird noch einmal mit Wasser ausgeschüttelt und dann mit Na₂SO₄ getrocknet. Das Lösungsmittel wird unter Vakuum verdampft und entfernt. Nach Trocknung werden 140,25 g leicht gelblich gefärbtes Produkt erhalten (Ausbeute 98,3%). Die gewonnene Substanz hat einen Schmelzpunkt von 123-124,5°C und ist chromatographisch homogen. Durch Kristallisation von 4,5 g Substanz aus 37,5 ml Ethylalkohol werden 3,75 g helleres Produkt gewonnen mit einem Schmelzpunkt von 125-126 °C. α_{D}²⁰: 27.7 (c = 2, CHCl₃). 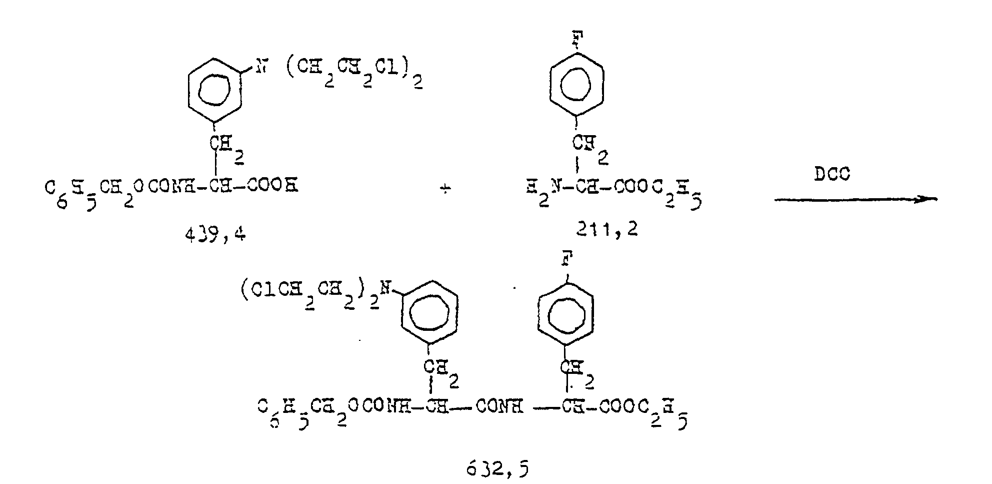 Analyse für C₃₂H₃₆Cl₂FN₃O₅
N% = 6,67 (berechnet 6,66
Cl% = 11,5 (berechnet = 11,2)

### b) L-m-Sarcolysyl-L-p-fluorphenylalanin-ethylester

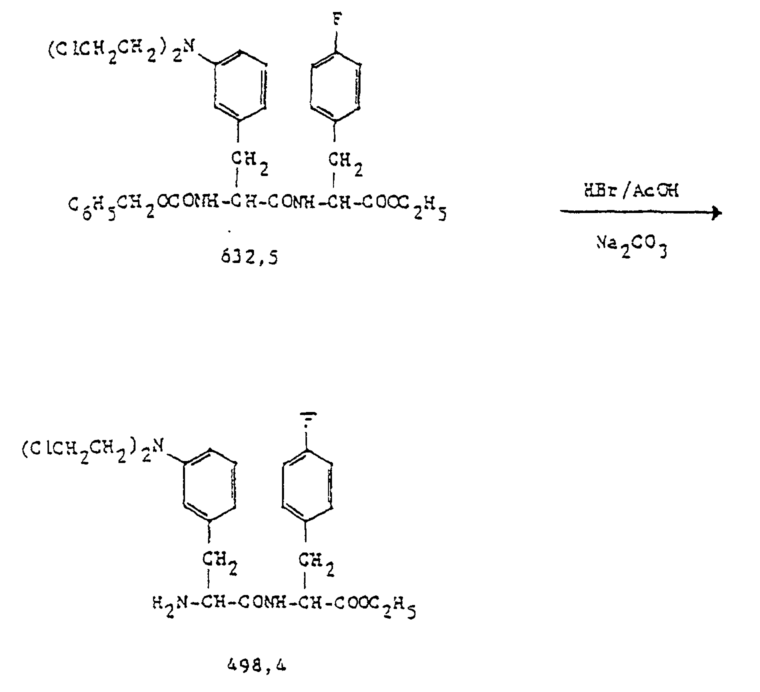

Unter Ausschluss der Luftfeuchtigkeit werden zu 390 g (0, 616 mol) N-Carbobenzoxy-L-m-sarcolysyl-L-p-fluorphenylalanin-ethylester unter langsamem Rühren 600 ml HBr in Eisessig (33 %) zugegeben. Die Auflösung und das Aufhören der CO₂-Entwicklung findet nach 40 Minuten statt. Es wird während weiteren 20 Minuten unter Rühren stehengelassen und mit ca. 400 ml Ether verdünnt. Man giesst das gesamte in 5 I Ether, welcher unter ständigem Rühren gehalten wird, dekantiert und wäscht das ausgefallene Oel 2 x mit 2 l Ether unter Dekantieren. Das Oel wird unter Rühren mit 4 l Wasser behandelt und man erhält einen Feststoff, welcher nach ca. 30 min. durch Filtration gesammelt wird und vollständig mit insgesamt 1500 ml Wasser und 500 ml Ether gewaschen wird. Das so erhaltene Bromhydrat wird in 2 I Ethylacetat suspendiert und unter Rühren mit 450 ml gesättigter Natriumcarbonatlösung behandelt, derart bis die Lösung alkalisch ist. Nachdem die Auflösung stattgefunden hat, filtriert man auf der Nutsche, um den supendierten Dicylohexylharnstoff (sehr wenig) zu entfernen. In einem Scheidetrichter trennt man die organische Schicht von der wässerigen Phase ab, und die wässrige Phase wird mit weiteren 500 ml Ethylacetat extrahiert. Die gereinigten Extrakte werden mit 300 ml Wasser gewaschen, Na₂CO₄ getrocknet und mit Norit behandelt. Es wird filtriert und das Filtrat wird unter dem Vakuum getrocknet (40°C). Der Rückstand wird noch vor seiner Festigung in 500 bis 1000 ml Ether aufgenommen. Aus der erhaltenen Lösung wird während der Nacht ein weisses Produkt ausgefällt. Ausbeute: 247 g (80,4 %) Smp. 100-102 °C.
α _{D}²⁰ = -7,5° (c=2, Chloroform)
TLC (BuOH/AcOH/H₂O 65:15:25; KMnO₄ verdünnt):
Eine Bande, Rf = 0,74
Analyse für C₂₄H₃₀Cl₂FN₃O₃
N% = 8,34 (berechnet 8,43)
Cl% = 14,1 (berechnet 14,2)

### c) N-Carbobenzoxy-L-prolyl-L-m-sarcolysyl-L-p-fluorphenylalaninethylester

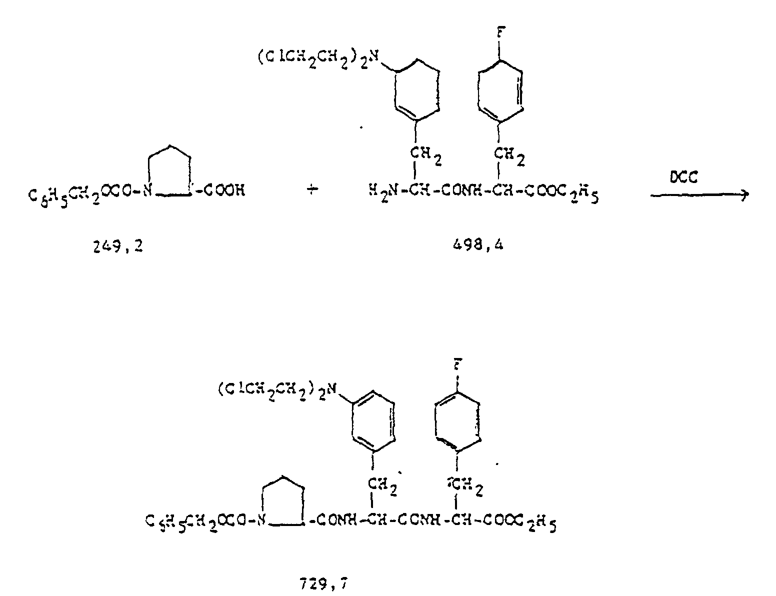

Eine Mischung von 249 g (0,5 mol) L-m-sarcolysyl-L-p-fluorphenylaianinethylester, 125 g (0,5 mol) N-Cbzo-L-Prolin und 109 g (0,525 mol) DCC in 3000 ml Chloroform wird während 30 Minuten unter Rühren stehen gelassen, mit externer Kühlung während weiteren 90 Minuten bei Zimmertemperatur (TLC, Silikagel G, Chf/Me₂CO 9:1; oder mit BuOH/AcOH/H₂O 65:15:25; KMnO₄, verdünnt, sauer). Nach der Entfernung des Dicyclohexylharnstoff durch Filtration wird das Lösungsmittel unter Vakuum abgedampft und der Rückstand wird noch in flüssigem Zustand in 800 ml Ether gegossen. Von der erhaltenen Lösung fällt langsam das Produkt aus, welches auf einem Filter gesammelt wird. Ausbeute 290 g (78,5%).
Smp. = 148-150°C, α_{D}²⁰ = -42,4° (c=2; Chloroform)
Analyse für C₃₇H₄₃FCl₂N₄O₆
N% = 7,78% (berechnet 7,68)
Cl% = 9,6 (berechnet 9,7)

### d) L-Prolyl-L-m-sarcolysyl-L-p-fluornhenylalanin-ethylester-hydrochlorid

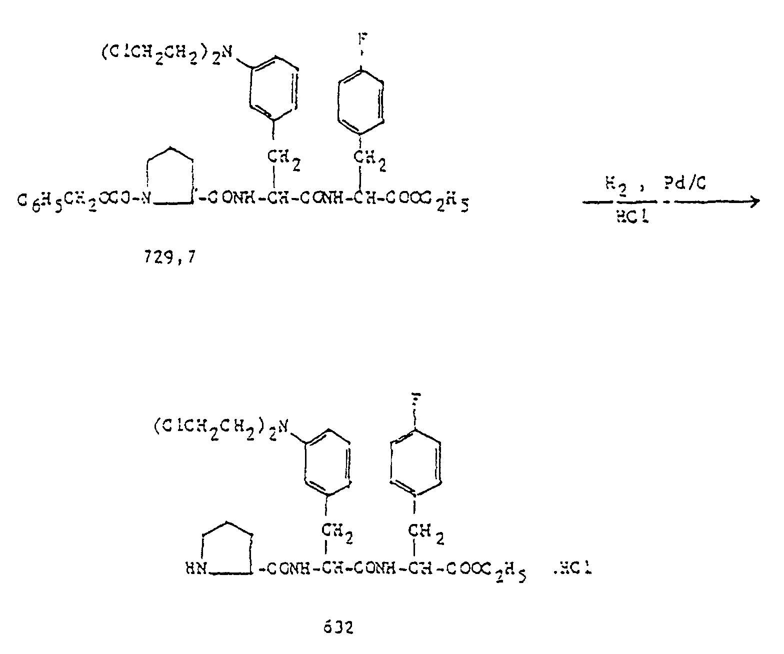

Eine Mischung von 157,5 (0,261 mol) N-Carbobenzoxy-L-prolyl-L-m-sarcolysyl-L-p-fluorphenylalanin-ethylester und 30 g Palladium auf Kohlenstoff 5% wird suspendiert unter einem Stickstoffstrom in 15 ml Eisessig und 1750 ml Metnanol. Die Reaktionsmischung wird unter Rühren gehalten und wird unter einem Wasserstoffstrom reduziert. Nach der Beendigung der CO₂-Entwicklung (nach 4 -5 Stunden) wird eine TLC-Chromatographiekontroile durchgeführt (Kieselgel G), wobei mit Chloroform-Aceton 9:1 eluiert wird und mit verdünntem KMnO₄ sichtbar gemacht wird.

Nach der Entfernung des Katalysators durch Filtration wird das Fiitrat mit konzentrierter ethanolischer HCI in stöchiometrischer Menge oder wenig mehr angesäuert. Der weisse, kristalline Niederschlag, welcher sich langsam bildet, wird auf einem Filter gesammeit und mit Ethanol oder mit Ether gewaschen: 85 g. Das Filtrat wird praktisch bis zur Trockenheit konzentriert und der Rückstand wird aus Ethanol umkristallisiert: 25 g. Vollständige Ausbeute: 110 g (80, 5%); Smp. 122 - 124 °C (Änderung des Aggregatszustandes)
α _{D}²⁰ =13,0°±0,5(c=2; MeOH)
TLC (Kieseigel G; BuOH/AcOH/H₂O 65:15:25; KMnO₄ verdünnt: eine Bande Rf = 0,54.
Analyse für C₂₉H₃₈Cl₃FN₄O₄
N % = 8,93% (berechnet 8,86)
Cl % = 16,7 % (berechnet 16,8)
Cl-%= 5,65% (berechnet 5,6)

### Beispiel 1:

Parenterales Präparat zur Behandlung von Melanomen.

| | Komponente | Menge |
|---|---|---|
| Peptid | PSF-ethylester · HCl | 8 g |
| Cyclodextrin-Trager | Hydroxypropyl-β-cyclodextrin | 16 g |
| andere Wirk- und Hilfsstoffe | Chlorphenamin Wasser, steril | 32 mg ad 100 ml |

- Darreichungsform:: sterile Lösung in Ampulle
- Dosiseinheit:: 40 mg Peptid/0,5 ml Lösung,
80 mg Hydroxypropyl-β-cyclodextrin /0,5 ml Lösung,
1,6 mg Chlorphenamin /0,5 ml Lösung

Bemerkungen: zur i.v. Verabreichung oder für Infusionen bestimmt.

### Beispiel 2:

Orales Zytostatikum in Kapsel

| | Komponente | Menge (Dosiseinheit) |
|---|---|---|
| Peptid | PSF-ethylester HCl | 12 mg |
| Cyclodextrin-Trager | β-Cyclodextrin | 25 g |

## Patentansprüche

1. Pharmazeutische Zusammensetzung zur Behandlung von Krebsleiden enthaltend als aktive Komponente mindestens eine Peptidverbindung, welche verzögert freigesetzt wird, **dadurch gekennzeichnet, dass** die Peptidverbindung ausgewählt ist aus der Gruppe:
- L-Seryl-L-p-fluorphenylalanyl-L-m-sarcolysin
- L-Prolyl-L-m-sarcolysyl-L-p-fluorphenylalanin
- L-m-Sarcolysyl-N-nitro-L-arginyl-L-norvalin
- L-p-Fiuorphenylalanyl-L-m-sarcolysyl-L-asparagin
- L-p-Fluorphenylalanyl-glycyl-L-m-sarcolysyl-norvalin
- L-m-Sarcolysyl-L-arginyl-L-lysyl-L-m-sarcolysyl-L-histidin
und Niederalkylester und/oder Säureadditionssalze davon, und dass die Zusammensetzung mindestens ein gegebenenfalls substituiertes Cyclodextrin als Hilfs- oder Trägersubstanz enthält.

2. Pharmazeutische Zusammensetzung nach Anspruch 1, **dadurch gekennzeichnet, dass** die Niederalkylester Methyl- oder Ethylester sind

3. Pharmazeutische Zusammensetzung nach Anspruch 1 oder 2 zur parenteralen Applikation, **dadurch gekennzeichnet, dass** das gegebenenfalls substitutierte Cyclodextrin Hydroxypropyl-β-cyclodextrin ist.

4. Pharmazeutische Zusammensetzung nach einem der Ansprüche 1 - 3 zur oralen Applikation, **dadurch gekennzeichnet, dass** das gegebenenfalls substituierte Cyclodextrin ausgewählt ist aus α-, β-, und γ-Cyclodextrin.

5. Pharmazeutische Zusammensetzung nach einem der Ansprüche 1 - 4, **dadurch gekennzeichnet, dass** die Peptidverbindung L-Prolyl-L-m-sarcolysyl-L-p-fluorphenylalanin ist, vorzugsweise in Form des Hydrochlorids des Ethylesters.

6. Zusammensetzung nach einem der Ansprüche 1 - 5, **dadurch gekennzeichnet, dass** das Mol-Verhältnis der Peptidverbindung zum gegebenenfalls substitutierten Cyclodextrin 1:1 bis 1:10 und vorzugsweise 1:2 bis 1:4 beträgt.

7. Zusammensetzung nach einem der Ansprüche 1 - 6, **dadurch gekennzeichnet, dass** sie zusätzlich noch mindestens einen pharmakologisch aktiven Wirkstoff enthält.

8. Zusammensetzung nach Anspruch 7, **dadurch gekennzeichnet, dass** der zusätzliche pharmakologisch aktive Wirkstoff Chlorphenamin ist.

## Claims

1. Pharmaceutical composition for the treatment of cancers containing as active component at least one peptide compound which is released with delay, **characterised In that** the peptide compound is selected from the group;
- L-seryl-L-p-fluorophenylalanyl-L-m-sarcolysine
- L-prolyl-L-m-sarcolysyl-L-p-fluorophenylalanine
- L-m-sarcolysyl-N-nitro-L-arginyl-L-norvaline
- L-p-fluorophenylalanyl-L-m-sarolysyl-L-asparagine
- L-p-fluorophenylalanyl-glycyl-L-m-sarcolysyl-norvaline
- L-m-sarcolysyl-L-arginyl-L-lysyl-L-m-sarcolysyl-L-histidine
and lower alkyl esters and/or add addition salts thereof, and that the composition contains at least one optionally substituted cyclodextrin as auxiliary or carrier substance.

2. Pharmaceutical composition according to claim 1, **characterised in that** the lower alkyl esters are methyl or ethyl esters.

3. Pharmaceutical composition according to claim 1 or 2 for parenteral application, **characterised in that** the optionally substituted cyclodextrin is hydroxypropyl-β-cyclodextrin.

4. Pharmaceutical composition according to one of the claims 1 - 3 for oral application, **characterised in that** the optionally substituted cydodextrin is selected from α-, β-, and γ-cyclodextrin.

5. Pharmaceutical composition according to one of the claims 1 - 4, **characterised in that** the peptide compound is L-prolyl-L-m-sarcolysyl-L-p-fluorophenylalanine, preferably in the form of the hydrochloride of the ethyl ester.

6. Composition according to one of the daims 1 - 5, **characterised in that** the molar ratio of the peptide compound to the optionally substituted cyclodextrin is from 1:1 to 1:10 and preferably from 1:2 to 1:4.

7. Composition according to one of the daims 1 - 6, **characterised in that** additionally it further contains at least one pharmacologically active substance.

8. Composition according to claim 7, **characterised in that** the additional pharmacologically active substance is chlorophenamine.

## Revendications

1. Composition pharmaceutique destinée au traitement de pathologies cancéreuses, contenant en tant que principe actif au moins un composé peptidique à libération retardée, **caractérisée en ce que** le composé peptidique est choisi dans l'ensemble :
- L-propyl-L-m-sarcolysyl-L-p-fluorophénylalanine
- L-m-sarcolysyl-N-nitro-L-arginyl-L-norvaline
- L-séryl-L-p-fluorophénylalanyl-L-m-sarcolysine
- L-p-fluorophénylalanyl-L-m-sarcolysyl-L-asparagine
- L-p-fluorophénylalanyl-glycyl-L-m-sarcolysylnorvaline
- L-m-sarcolysyl-L-arginyl-L-lysyl-L-m-sarcolysyl-L-histidine
et les esters alkyliques inférieurs et/ou sels d'addition avec un acide de ceux-ci, et **en ce que** la composition contient, en tant qu'adjuvant ou excipient, au moins une cyclodextrine éventuellement substituée.

2. Composition pharmaceutique selon la revendication 1, **caractérisée en ce que** les esters alkyliques inférieurs sont des esters méthyliques ou éthyliques.

3. Composition pharmaceutique selon la revendication 1 ou 2 pour administration parentérale, **caractérisée en ce que** la cyclodextrine éventuellement substituée est l'hydroxypropyl-β-cyclodextrine.

4. Composition pharmaceutique selon l'une des revendications 1 à 3 pour administration orale, **caractérisée en ce que** la cyclodextrine éventuellement substituée est choisie parmi les cyclodextrines α, β et γ.

5. Composition pharmaceutique selon l'une des revendications 1 à 4, **caractérisée en ce que** le composé peptidique est la L-propyl-L-m-sarcolysyl-L-p-fluorophénylalanine, de préférence sous forme du chlorhydrate de l'ester éthylique.

6. Composition selon l'une des revendications 1 à 5, **caractérisée en ce que** le rapport en moles du composé peptidique à la cyclodextrine éventuellement substituée est de 1:1 à 1:10 et de préférence de 1:2 à 1:4.

7. Composition selon l'une des revendications 1 à 6, **caractérisée en ce qu'**elle contient en outre encore au moins un principe actif pharmacologique.

8. Composition selon la revendication 7, **caractérisée en ce que** le principe actif pharmacologique supplémentaire est le chlorphénamine.
